# EUROPEAN PATENT APPLICATION

(11) **EP 3 326 625 A1**
(43) Date of publication of application: **30.05.2018**
(21) Application number: 17163492.6
(22) Date of filing: 06.07.2012
(51) Int. Cl.: A61K 31/192, A61K 31/352, A61K 31/353, A61K 31/385, A61K 36/82, A61K 45/06, A61P 35/00

(54) **SYSTEMS, METHODS, AND FORMULATIONS FOR TREATING CANCER**

(30) Priority: 07.07.2011 US 201161505393 P; 14.07.2011 US 201161507950 P
(62) Divisional of application: 12806902.8
(71) Applicant: Research Cancer Institute of America, Fresno, California 93720 (US)
(72) Inventor: NEZAMI, Mohammad, MD, Clovis, CA California 93649 (US)
(74) Representative: Zacco Sweden AB

(57) **Abstract**

A method and compositions for treating cancer is described using at least two epigenetic modifiers. In various embodiments, hyperbaric oxygen therapy and glycolytic inhibition therapy are used as well.

## Description

### CROSS REFERENCE TO RELATED APPLICATIONS

This application claims the benefit of U.S. Provisional Application No. 61/505,393, filed July 7, 2011, and entitled "TARGETED INTRAVENOUS THERAPIES FOR PATIENTS WITH IMMUNE DYSFUNCTION AND CANCER," which is hereby incorporated by reference in its entirety, and U.S. Provisional Application No. 61/507,950, filed July 14, 2011, and entitled "METHOD FOR TREATING CANCER," which is hereby incorporated by reference in its entirety.

### FIELD

The present disclosure relates to various systems, methods, and formulations for the treatment of patients with cancer, and in particular, patients having cancer in advanced stages.

### BACKGROUND

In 2007, the ten most commonly diagnosed cancers among men in the United States included cancers of the prostate, lung, colon, rectum, and bladder; melanomas of the skin; non-Hodgkin's lymphoma; kidney cancer, mouth and throat cancer, leukemia, and pancreatic cancer. In women, the most common cancers were reported as breast, lung and colon cancer. Overall, 758,587 men were told they had cancer and 292,853 men died from cancer in the U.S. in 2007. In women, there has been a prevalence of 6,451,737 advanced cases reported in 2008 by the Surveillance, Epidemiology, and End Results (SEER) Program of the National Cancer Institute. In general there were 11,957,599 advanced cancer cases in the US reported in 2010 by the Centers for Disease Control and Prevention (CDC) and the incidence has been almost unchanged over the previous 8 years (482,000 cases in 2000 versus 456,000 cases in 2008). There has been an annual change of only approximately 0.6% in cancer incidence between the years of 1999 to 2008. Statistics show that deaths caused by advanced cancers from all types have not significantly changed since a decade ago, and in some cases, such as lung cancer, the death rate is rising, especially among women. Even as more chemotherapy agents are introduced to the market for advanced stages of disease, the patient survival rates have remained essentially unchanged. Moreover, the potential toxicity of many chemotherapeutic agents can be a devastating factor both for the clinician and the patient. Therefore, the need for non-toxic therapies, used either alone or in combination with traditional chemotherapy, is evident.

### SUMMARY

In various embodiments, a pharmaceutical formulation for prophylaxis or treatment of cancer is provided comprising two or more "epigenetic modifiers". In further embodiments, the epigenetic modifiers may be histone deacetylase inhibitor and/or demethylating agents. In yet further preferred embodiments, the demethylating agents can indirectly function as histone deacetylase inhibitors (HDACI) and vice versa. In other particularly preferred embodiments, the epigenetic modifiers may be selected from a group comprising sodium phenyl butyrate (SDB), lipoic acid (LA), quercetin, valproic acid, hydralazine, bactrim, green tea extract (e.g., epigallocatechin gallate (EGCG)), curcumin, sulforphane and allicin/ diallyl disulfide. In a preferred embodiment, one epigenetic modifier comprises sodium phenyl butyrate (SPB) and a second epigenetic modifier comprises quercetin. In another preferred embodiment, one epigenetic modifier comprises sodium phenyl butyrate (SPB) and a second epigenetic modifier comprises green tea extract, such as epigallocatechin gallate (EGCG).

In other embodiments, the pharmaceutical formulation may further comprise one or more glycolytic inhibitors. In further embodiments, the glycolytic inhibitors may be selected from a group comprising dichloroacetic acid, octreotide, and 2 deoxy glucose (2DG). In yet other embodiments, the pharmaceutical formulation may further comprise one or more oxidants or antioxidants. In further embodiments the oxidants or antioxidants are selected from a group comprising vitamin C, germanium, L carnitine, taurine, gluthatione, lysine, proline, hydrogen peroxide (H2O2), and dimethyl sulfoxide (DMSO).

In various embodiments, a unit dose of a pharmaceutical formulation for prophylaxis or treatment of cancer is provided comprising the two or more epigenetic modifiers in a combined form, wherein the epigenetic modifiers are present in a dosage sufficient to cause tumor response in a human (e.g., decreased tumor markers, shrinkage of a human tumor) as measured by laboratory and/or radiologic studies after administration of between about 1 unit doses and about 60 unit doses. In other various embodiments, a unit dose of a pharmaceutical formulation for prophylaxis or treatment of cancer is provided comprising two or more epigenetic modifiers in a combined form, wherein the epigenetic modifiers are present in a dosage sufficient to cause increase in immune system measured by increase in white blood count (WBC) and/ or natural killer (NK) cell activity in a human after administration of between about 1 unit dose and about 60 unit doses.

In still further embodiments, a kit is provided comprising a unit dose of a pharmaceutical formulation for prophylaxis or treatment of cancer comprising two or more epigenetic modifiers, and a container wherein the unit dose is at least partially contained.

In various embodiments, a method of prophylaxis or treatment is provided comprising administering therapeutically effective amounts of two or more epigenetic modifiers to an animal. In a one embodiment, the prophylaxis or treatment is for cancer. In a preferred embodiment, the cancer is an epigenetically driven cancer. In another particularly preferred embodiment, the selected from a group comprising gliomas, colon cancer, pancreatic cancer, leukemia, non small cell lung carcinoma, and malignant melanoma. In another preferred embodiment, the cancer is hypoxic.

In another embodiment, a method of prophylaxis or treatment is provided comprising administering therapeutic amounts of one or more epigenetic modifiers to cancer cell lines or culture invitro or invivo in an animal model and subjecting the animal to hyperbaric oxygen environment. In one particular embodiment, the one or more epigenetic modifiers may be administered before the subjecting of the animal to hyperbaric oxygen. In an alternate particular embodiment, the one or more epigenetic may be administered after the subjecting of the animal to hyperbaric oxygen. In yet another alternate particular embodiment, the one or more epigenetic modifiers may be administered before and after the subjecting of the animal to hyperbaric oxygen environment. In another embodiment, the epigenetic modifiers may be administered separately or in a combined form. In a particularly preferred embodiment, the subjecting occurs within about 24 hours before or after the administering. In a second preferred embodiment, the subjecting occurs between about five (5) minutes and about ninety (90) minutes before or after the administering. In third preferred embodiment, the subjecting occurs for between about thirty (30) minutes and about three (3) hours before or after the administering.

In another embodiment, for any of the foregoing methods, chemotherapy or radiation may be one or more additional steps that occur before or after administering any epigenetic modifiers, whether in single or combined form, separately or mixed, before or after the subjecting of the animal to a hyperbaric oxygen environment.

In various embodiments, a method of prophylaxis or treatment is provided comprising administering therapeutically effective amounts of one or more glycolytic inhibitors to modifiers to cancer cell lines or culture invitro or invivo in an animal model, and subjecting the animal to a hyperbaric oxygen environment. In a particular embodiment, the subjecting occurs within about twenty-four (24) hours before or after the administering. In a second particular embodiment, the subjecting occurs between about five (5) minutes and about ninety (90) minutes before or after the administering. In another particularly preferred embodiment, the subjecting occurs for between about thirty (30) minutes and about three (3) hours before or after the administering.

In further embodiments, a method of prophylaxis or treatment comprising administering therapeutically effective amounts of one or more glycolytic inhibitors to modifiers to cancer cell lines or culture invitro or invivo in an animal model and administering therapeutically effective amounts of one or more epigenetic modifiers to the animal. In a particular embodiment, the one or more glycolytic administrations and one or more epigenetic modifier administrations occur within about twenty-four (24) hours before or after each other. In a second particular embodiment, the one or more glycolytic inhibitor administrations occurs between about five (5) minutes and about ninety (90) minutes before or after administering the one or more epigenetic modifier administrations. In a third particular embodiment, the one or more glycolytic inhibitor administrations occurs between about thirty (30) minutes and about three (3) hours after the one or more epigenetic modifier administrations.

### DETAILED DESCRIPTION

The below specification, examples, and data provide a complete description of the manufacture and use of the composition of the invention. Since many embodiments of the invention can be made without departing from the spirit and scope of the invention, the invention resides in the claims hereinafter appended. The examples set forth above are provided to give those of ordinary skill in the art with a complete disclosure and description of how to make and use the preferred embodiments of the compositions and the methods, and are not intended to limit the scope of what the inventors regard as their invention. Modifications of the above-described modes for carrying out the invention that are obvious to persons of skill in the art are intended to be within the scope of the following claims. Unless otherwise specified, the following definitions apply.

### DEFINITIONS

The term "administer," "administration," "deliver" or "delivery" (collectively "administration"), as used herein, means administration to the body via tablets, capsules, softgel capsules, intravenous, intramuscular, and/or subcutaneous injections, transdermal patches, creams, gels, or other mechanisms known in the art or hereinafter developed.

The term "active ingredient," as used herein, may refer to any material that is or is intended to be biologically active.

The term "combined form," as used herein, may refer to the presence of two or more active ingredients in the same medium. For example, if active ingredient A and active ingredient B are both in the same saline medium, the mixture of active ingredient A and active ingredient B may be said to be in combined form.

The term "epigenetic modifier," as used herein, may refer to a material that affects, is believed to affect, or tends to affect gene expression and function. Also as used herein, "epigenetically driven" may refer to any material that is affected by, or tended to be affected by, gene expression and function.

The term "glycolytic inhibitor," as used herein, may refer to a material that inhibits, is believed to inhibit, or tends to inhibit glycolysis from occurring in a cancerous cell.

The term "saline," as used herein, may refer to a sterile or substantially sterile sodium chloride in water solution. Saline may be suitable for intravenous injection into a human.

The term "unit dose," as used herein, may refer to a mass or volume of pharmaceutical formulation intended to be given to a patient at one time.

### DESCRIPTION

It has been found that use of two or more epigenetic modifiers in the treatment of cancer produces unexpected and synergistic therapeutic results. In particular, co-administration, co-formulation, and/or temporally closely spaced administration tend to yield particularly effective results. Moreover, in various embodiments, hyperbaric oxygen treatment further enhances therapeutic effect. It has also been found that various antioxidants and glycolytic inhibitors may be beneficially used in addition to the two or more epigenetic modifiers. The co-administration, co-formulation and/or temporally closely spaced administration of any of the epigenetic modifiers, antioxidants, glycolytic inhibitors and/or hyperbaric oxygen treatment may occur in any order, but may occur within about 24 hours before or after each other, between about five (5) minutes and about ninety (90) minutes before or after each other, or between about thirty (30) minutes and about three (3) hours before or after each other.

Formulations in accordance with various embodiments comprise two or more epigenetic modifiers. Epigenetic modifiers may work together to alter genetic expression in cancerous cells and precancerous cells. Epigenetic modifiers may be selected to increase or decrease genetic expression. For example, epigenetic modifiers may be used to reduce expression of rat sarcoma ("Ras") family genes and/or b-cell lymphoma 2 ("bcl-2") genes.

Epigenetic modifiers found in various embodiments include, but are not limited to, histone deacetylase inhibitors and demythylating agents. Demethylating agents are agents that inhibit or tend to inhibit the methylation of DNA and/or histones by inhibiting or tending to inhibit methylation enzymes, such as DNA methyltrasferases (DNMT) or histone methyltransferases. Demythylating chemotherapy agents include, but are not limited to cytidine analogs, such as 5-azacytidine (azacitidine) and 5-azadeoxycytidine (decitabine). HDACIs, in particular, are known to interfere with or tend to interfere with histone deacetylases. Histone deacetylase is an enzyme that removes acetyl groups from histones and is active prior to transcription. Among other characteristics, some epigenetic modifiers have also been shown to inhibit angiogenesis.

In various embodiments, although any epigenetic modifier is contemplated for use with the present invention, the HDACIs may be direct (e.g., act by direct association with target enzymes or indirect (e.g., act by indirect means, such as change in chromatin shape). In addition, HDACI may comprise one or more of sodium phenylbutyrate ("SPB"), lipoic acid ("LA"), quercetin, valproic acid, hydralazine, bactrim, green tea extract (e.g., epigallocatechin gallate (EGCG)), curcumin, sulforphane and allicin/ diallyl disulfide.

SPB is currently classified by the FDA as an orphan drug for the treatment of urea cycle disorders. Phenylbutyrate ("PB") is a prodrug. In the human body, PB is metabolized by beta-oxidation to phenylacetate. Phenylacetate conjugates with glutamine to form phenylacetylglutamine, which is ultimately eliminated in urine. Phenylbutyric acid ("PBA") has growth inhibitory and differentiation-inducing activity in vitro and in vivo in model systems. Although not bound by this theory, PBA is believed to stop the cell cycle in its G1-G0 phase. PB is an efficient HDACI and is believed to induce apoptosis via c-jun N-terminal kinase ("JNK"). In lung carcinoma cells, 56 p21waf1-mediated growth arrest in MCF-7 cells, tumor necrosis factor (TNF)-α58 or peroxisome proliferator-activated receptor (PPAR)λ-mediated cell differentiation, and is more potent than phenylacetate in prostate cancer cells, while increasing MHC class I expression. PB is converted in vivo into the active metabolite phenylacetate ("PA") by β-oxidation in the liver and kidney mitochondria. Most dose-limiting toxicities are fatigue, nausea, and somnolence. Preliminary studies have been conducted in patients with recurrent glioblastoma multiforme. It is believed that SPB works by affecting the NF Kappa- B pathway, lowering the inflammatory response, and down regulating more than a hundred genes.

Toxicity studies of SPB have shown that oral doses up to 36 grams per day demonstrate minimal toxicity. In one study, 25 percent of patients had stable disease for more than 6 months while on the drug. SPB in oral form is well tolerated and achieves the concentration in vivo that has been shown to have biological activity in vitro. It has been suggested that SPB has a role as a cytostatic agent. However in most studies SPB has been used orally and not intravenously.

In various embodiments, quercetin is used as an HDACI. Quercetin is also used as a cancer stem cell differentiator as well as blocker for many pathways and signaling molecules as well as chemosensitizer as well as apoptotic agent. Quercetin is a polyphenyl extracted from apples. Several mechanisms have been suggested to show quercetin's anticancer effects. It has been suggested that quercetin may interact with a variety of cellular receptors, and that quercetin inhibits cellular growth phase at G1 and G2, inhibits tyrosine kinase to prevent uncontrolled proliferation, influences estrogen receptors, and interacts with heat shock proteins to prevent proliferation.

It has also been shown that quercetin may interact with receptors like Raf and MEK that are involved in tumor proliferation. Interactions with other receptors, such as cell surface receptors, are also suspected. In addition, it is believed that quercetin may act as a modifier of signal transduction. Quercetin is reported to affect cell cycle regulation, cell death, inflammatory reactions and derivation of new blood supply.

Toxicity studies have been conducted on quercetin. An open label, uncontrolled dose-finding clinical trial of quercetin was conducted. In the trial, increasing values of up to 1700mg/m2 intravenous quercetin were administered for about 3 weeks to 50 patients who had cancer deemed no longer treatable by conventional methods. Patients with a variety of cancers were treated including large bowel, stomach, pancreas, ovarian and melanoma. None of the patients achieved suppression as defined by the radiological criteria of WHO, but two showed sustained decreases in unique cancer markers following quercetin therapy (one with metastatic hepatocellular carcinoma, and the other with stage 4 metastatic ovarian cancer that had been previously unresponsive to chemotherapy). In addition, tyrosine kinase levels were measured in 11 subjects, and a decrease in 9 was reported. Tyrosine kinase is often studied in oncology as it may lead to the uncontrolled proliferation of cancer by overriding signals that control cell growth. As a result, it was concluded that quercetin may have ability to inhibit tyrosine kinase, and further study should be undertaken at doses no higher than 1400 mg/m2. The results of this study have been supported by several in vitro trials, in which quercetin caused suppression of tyrosine kinase expression in malignant and non-malignant cells.

While not being bound by this theory, it is hypothesized that quercetin can show promising results in treating almost every cancer cell due to its genetic regulatory effects including, for example, decreasing genetic expression of the RAS genes and bcl-2 genes. It has also been suggested that quercetin has a preventive role in cancer incidence. Quercetin intake was negatively correlated with pancreatic cancer among current smokers, showing a significantly decreased (0.55) relative risk between the highest and lowest quintiles of intake.

There do not, however, appear to be human studies that have looked at quercetin's effects when used intravenously, in conjunction with other epigenetic therapies.

In various embodiments, lipoic acid ("LA") is used as a HDACI. Lipoic acid is also a topoisomerase inhibitor and an oxidative agent for glycolytic therapy. At low levels, LA is a cofactor of pyrovate dehydrogenase in mitochondria. LA is not synthetized in human being and is not available in enough quantities in diet or food. Naturally occurring LA may not be immediately available from dietary sources. Low levels of LA have been correlated to a variety of disease states. LA is generally considered safe and non-toxic.

More recently, it is believed that the primary effect of LA is as an inducer of the oxidative stress response. In that regard, LA may be effective with hyperbaric oxygen treatment by potentiating the oxidation in combination therapy against cancer. It has been shown that alpha-lipoic acid induces apoptosis in human colon cancer cells by increasing mitochondrial respiration with a concomitant free oxygen radical generation. Several studies provide evidence that alpha lipoic acid can effectively induce apoptosis in human colon cancer cells by a pro-oxidant mechanism that is initiated by an increased uptake of oxidizable substrates into mitochondria.

Recent studies have shown promise in using LA to treat a variety of cancer cells in mouse cancer models: MBT-2 bladder transitional cell carcinoma, B16-F10 melanoma and LL/2 Lewis lung carcinoma. It is believe that LA decreases cancer cell viability and increases DNA fragmentation of the cells. In general, LA's anticancer effect appears to be mediated by inducing apoptosis through caspase-independent and caspase-dependent pathways, which is mediated by intracellular Ca2+. LA is generally considered safe and non-toxic. Alpha-lipoic acid is approved in Germany as a drug for the treatment of polyneuropathies, such as diabetic and alcoholic polyneuropathies, and liver disease.

Green tea extract, such as Epigallocatechin gallate (EGCG), also known as epigallocatechin 3-gallate, is also contemplated for use in the present invention. EGCG is the ester of epigallocatechin and gallic acid, and is a type of catechin. EGCG is the most abundant catechin in tea and is a potent antioxidant that may have therapeutic applications in the treatment of many disorders (e.g. cancer). It is generally found in green tea but not black tea; during black tea production, the catechins are converted to theaflavins and thearubigins. EGCG can be found in many supplements.

There is increasing evidence to show that EGCG - along with other flavonoids - can be beneficial in treating certain cancers, including brain, prostate, cervical and bladder cancers. EGCG has been shown to bind and inhibit the anti-apoptotic protein Bcl-xl, which has been implicated in both cancer cell and normal cell survival. Also, EGCG was, among other tea polyphenols, found to be a strong topoisomerase inhibitor, similar to some chemotherapeutic anticancer drugs, for example, etoposide and doxorubicin.

Formulations according to various embodiments comprising one or more HDACIs may be in a combined form.

Formulations, systems and methods in accordance with various embodiments may further comprise one or more pharmaceutically acceptable excipients. Formulations may be administered alone or in combination with one or more other compounds disclosed herein or in combination with one or more other drugs (or as any combination thereof). Generally, formulations described herein will be administered as a formulation in association with one or more pharmaceutically acceptable excipients. The choice of excipient will to a large extent depend on factors such as the particular mode of administration, the effect of the excipient on solubility and stability, and the nature of the dosage form.

Pharmaceutical compositions suitable for the delivery of compounds of the present invention and methods for their preparation will be readily apparent to those skilled in the art. Such compositions and methods for their preparation may be found, for example, in Remington's Pharmaceutical Sciences, 19th Edition (Mack Publishing Company, 1995), which is incorporated herein in its entirety by reference.

Formulations in accordance with various embodiments may comprise any pharmaceutically acceptable carrier or diluent, such as saline. Normal saline may comprise sterile water and sodium chloride. For example, normal saline and/or D5 saline may be used. D5 saline comprises saline with 5% dextrose.

The formulations of the invention may also be administered directly into the blood stream, into muscle, or into an internal organ. Suitable means for parenteral administration include intravenous, intraarterial, intraperitoneal, intrathecal, intraventricular, intraurethral, intrasternal, intracranial, intramuscular and subcutaneous. Suitable devices for parenteral administration include needle (including microneedle) injectors, needle-free injectors and infusion techniques.

Parenteral formulations are typically aqueous solutions which may contain excipients such as salts, carbohydrates and buffering agents (preferably to a pH of from about 3 to about 11), but, for some applications, they may be more suitably formulated as a sterile non-aqueous solution or as a dried form to be used in conjunction with a suitable vehicle such as sterile, pyrogen-free water.

The preparation of parenteral formulations under sterile conditions, for example, by lyophilisation, may readily be accomplished using standard pharmaceutical techniques well known to those skilled in the art.

Pharmaceutically acceptable salts of the compounds disclosed herein include the acid addition and base salts thereof. Suitable acid addition salts are formed from acids which form non-toxic salts. Examples include, but are not limited to, the acetate, aspartate, benzoate, besylate, bicarbonate/carbonate, bisulphate/sulphate, borate, camsylate, citrate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulphate, naphthylate, 2-napsylate, nicotinate, nitrate, orotate, oxalate, palmitate, pamoate, phosphate/hydrogen phosphate/dihydrogen phosphate, saccharate, stearate, succinate, tartrate, tosylate and trifluoroacetate salts.

Suitable base salts are formed from bases which form non-toxic salts. Examples include the aluminum, arginine, benzathine, calcium, choline, diethylamine, diolamine, glycine, lysine, magnesium, meglumine, olamine, potassium, sodium, tromethamine and zinc salts.

Hemisalts of acids and bases may also be formed, for example, hemisulphate and hemicalcium salts.

Pharmaceutically acceptable salts of active ingredients as disclosed herein may be prepared by one or more of three methods (although any method of preparing pharmaceutically acceptable salt may be used):
(i) by reacting the compound of active ingredients as disclosed herein with the desired acid or base;
(ii) by removing an acid- or base-labile protecting group from a suitable precursor of the active ingredients as disclosed herein or by ring-opening a suitable cyclic precursor, for example, a lactone or lactam, using the desired acid or base; or
(iii) by converting one salt of the active ingredients as disclosed herein to another by reaction with an appropriate acid or base or by means of a suitable ion exchange column.

All three reactions are typically carried out in solution. The resulting salt may precipitate out and be collected by filtration or may be recovered by evaporation of the solvent. The degree of ionization in the resulting salt may vary from completely ionized to almost non-ionized. For a review on suitable salts, see Handbook of Pharmaceutical Salts: Properties, Selection, and Use by Stahl and Wermuth (Wiley-VCH, Weinheim, Germany, 2002), in which all applicable portions are incorporated herein by reference.

In various embodiments, two or more active ingredients, such as two or more HDACIs, may not be in a combined form but may be co-administered. Co-administration may comprise administration to a patient at the same time, or within a closely spaced time period. For example, co-administration may comprise administering SPB and quercetin at the same time. Also for example, co-administration may comprise administering SPB and quercetin within between about twenty-four (24) hours of one another, or about one (1) minute and about sixty (60) minutes of one another, or about five (5) minutes and about ninety (90) minutes one another, or about thirty (30) minutes and about three (3) hours one another.

In various embodiments, one or more oxidants or antioxidants may be present. Examples include, but are not limited to: vitamin C, germanium, L carnitine, taurine, gluthatione, lysine, proline, hydrogen peroxide (H2O2), and dimethyl sulfoxide (DMSO). Oxidants may be any chemical, substance, molecule or compound that releases or assists in the release of free radicals, resulting in damage to cells, including cancer cells. In general, oxidants (also called an oxidizing agent, oxidizer or oxidiser) remove electrons from another reactant in a redox chemical reaction. The oxidant is "reduced" by taking electrons onto itself and the reactant is "oxidized" by having its electrons taken away. Antioxidants may be any chemical, substance, molecule or compound that delays or prevents the oxidation of a substrate. In general, antioxidants reduce the rate of oxidation reactions, which are chemical reactions that involve the transfer of electrons from one substance to an oxidizing agent. Antioxidants may slow these reactions either by reacting with intermediates and halting the oxidation reaction directly, or by reacting with the oxidizing agent and preventing the oxidation reaction from occurring. The same substance could act as an oxidant or antioxidant under different circumstances or conditions. In particular, the dose of the substance may determine whether a substance acts as an oxidant or an antioxidant. By way of example. vitamin C in a dose of 25 gram IV has oxidative or oxidant properties, but at lower doses, vitamin C has antioxidant properties.

In various embodiments, one or more glycolytic inhibitors may be present. Examples include, but are not limited to: dichloroacetic acid, octreotide, and 2 deoxy glucose (2DG). However, not all glycolytic inhibitors have been found to be effective. In particular, although previous studies have utilized 3 bromopyruvate (as an alkylating agent and inhibitor glycosis) to target cancer cells, it was not found effective in or for use in the present invention. Generally, relating to glycolytic inhibitors, one strategy to destroy or prevent cancers is by targeting their cellular energy production factories. Nucleated human cells have two types of energy production units, i.e., systems that make the "high energy" compound ATP from ADP. One type is "glycolysis," the other the "mitochondria." Mitochondria are the major ATP producers (>90%) in non-cancerous cells. However, human cancers tend to rely on both mechanisms. Glycolysis may contribute nearly half the ATP even in the presence of oxygen (referred to as the "Warburg effect"). Thus, glycolytic inhibitors may be useful in the treatment of various cancers.

Dichloroacetic acid ("DCA") is a byproduct of chlorination of water. By stimulating the activity of pyruvate dehydrogenase, DCA facilitates oxidation of lactate and decreases morbidity in acquired and congenital forms of lactic acidosis. The dichloroacetate ion stimulates the activity of the enzyme pyruvate dehydrogenase by inhibiting the enzyme pyruvate dehydrogenase kinase. Thus, it decreases lactate production by shifting the metabolism of pyruvate from glycolysis towards oxidation in the mitochondria.

Cancer cells tend to change the way they metabolize oxygen in a way that promotes their survival. Solid tumors, including the aggressive primary brain cancer glioblastoma multiforme, develop resistance to cell death, in part as a result of a switch from mitochondrial oxidative phosphorylation to cytoplasmic glycolysis. DCA depolarizes mitochondria, increases mitochondrial reactive oxygen species, and induces apoptosis in glycolytic cancer cells, both in vitro and in vivo.

DCA therapy also inhibits the hypoxia-inducible factor-1alpha, promoted p53 activation, and suppressed angiogenesis both in vivo and in vitro. There is substantial evidence in preclinical in vitro and in vivo models that DCA might be beneficial in human cancer. Furthermore, activating mitochondria by DCA increases O2 consumption in the tumor and dramatically enhances the effectiveness of hypoxia-specific chemotherapies in animal models. In laboratory studies of isolated cancer cells grown in tissue culture, DCA restores the original metabolism, and promotes their self-destruction.

Octreotide (brand name SANDOSTATlN®) is an octapeptide that mimics natural somatostatin pharmacologically, though it is a more potent inhibitor of growth hormone, glucagon, and insulin than the natural hormone. Octreotide is absorbed quickly and completely after subcutaneous application. Maximal plasma concentration is reached after 30 minutes.

Oncogenes may express proteins of "Tyrosine kinase receptor pathways," a receptor family including insulin or IGF-Growth Hormone receptors. Other oncogenes alter the PP2A phosphatase brake over these kinases. Octreotide has been used in variety of medical conditions since 1979. Since it inhibits secretion of insulin and also acts as a suppressing agent for Insulin growth factor one (IgFI), it's use has been suggested in a variety of glycolytic cancers. Octreotide is found to have therapeutic application beneficial to patients as shown by experiments on animals.

GH hormone induces in the liver, the synthesis and release of insulin like growth factor (IGF). The latter, activates like insulin, the IGF-tyrosine kinase receptors (IGFR), triggering the MAP kinase-ERK mitogenic signal. In normal physiology GH stimulates a triglyceride lipase in adipocytes, increasing the release of fatty acids and their β oxidation. In parallel, GH would close the glycolytic source of acetyl CoA, perhaps inhibiting the hexokinase interaction with the mitochondria, This effect, which renders apoptosis possible, does not occur in tumor cells.

Inasmuch as it may desirable to administer a combination of active ingredients, for example, for the purpose of treating a particular disease or condition, it is within the scope of the present disclosure that two or more pharmaceutical formulations, may conveniently be combined in the form of a kit suitable for co-administration of the compositions.

Thus, a kit as disclosed herein comprises two or more separate pharmaceutical formulations, at least one of which contains an active ingredient as described herein, and means for separately retaining said formulations, such as a container, divided bottle, or divided foil packet. An example of such a kit is the familiar blister pack used for the packaging of tablets, capsules and the like.

The kit is particularly suitable for administering different dosage forms, for example, oral and parenteral, for administering the separate compositions at different dosage intervals, or for titrating the separate compositions against one another. To assist compliance, the kit typically comprises directions for administration and may be provided with a memory aid.

The compounds of the invention may exist in both unsolvated and solvated forms. The term 'solvate' is used herein to describe a molecular complex comprising the compound of the invention and a stoichiometric amount of one or more pharmaceutically acceptable solvent molecules, for example, ethanol. The term 'hydrate' is employed when said solvent is water.

Included within the scope of the invention are complexes such as clathrates, drug-host inclusion complexes wherein, in contrast to the aforementioned solvates, the drug and host are present in stoichiometric or non-stoichiometric amounts. Also included are complexes of the drug containing two or more organic and/or inorganic components, which may be in stoichiometric or non-stoichiometric amounts. The resulting complexes may be ionized, partially ionized, or non-ionized. For a review of such complexes, see J Pharm Sci, 64 (8), 1269-1288, by Haleblian (August 1975), which is incorporated herein in its entirety by reference.

Hereinafter all references to active ingredients as disclosed herein include references to salts, solvates and complexes thereof and to solvates and complexes of salts thereof.

The active ingredients as disclosed herein, including all polymorphs and crystal habits thereof, prodrugs and isomers thereof (including optical, geometric and tautomeric isomers) as hereinafter defined as active ingredients as disclosed herein. For example, all HDACIs and glycolytic inhibitors disclosed herein include all polymorphs and crystal habits thereof, prodrugs and isomers thereof (including optical, geometric and tautomeric isomers).

As indicated, so-called 'pro-drugs' of the active ingredients as disclosed herein are also within the scope of the invention. Thus certain derivatives of active ingredients as disclosed herein which may have little or no pharmacological activity themselves can, when administered into or onto the body, be converted into active ingredients as disclosed herein having the desired activity, for example, by hydrolytic cleavage. Such derivatives are referred to as 'prodrugs'. Further information on the use of prodrugs may be found in Prodrugs as Novel Delivery Systems, Vol. 14, ACS Symposium Series (T. Higuchi and W. Stella) and Bioreversible Carriers in Drug Design, Pergamon Press, 1987 (ed. E. B. Roche, American Pharmaceutical Association), which is incorporated herein in its entirety by reference.

Prodrugs in accordance with the present disclosure can, for example, be produced by replacing appropriate functionalities present in the active ingredients as disclosed herein with certain moieties known to those skilled in the art as 'pro-moieties' as described, for example, in Design of Prodrugs by H. Bundgaard (Elsevier, 1985), which is incorporated herein in its entirety by reference.

Examples of replacement groups in accordance with the foregoing examples and examples of other prodrug types may be found in the aforementioned references.

Also included within the scope of the invention are metabolites of active ingredients as disclosed herein, that is, compounds formed in vivo upon administration of the drug.

Compounds of active ingredients as disclosed herein may contain one or more asymmetric carbon atoms may exist as two or more stereoisomers. Where active ingredients as disclosed herein contains an alkenyl or alkenylene group, geometric cis/trans (or Z/E) isomers are possible. Where structural isomers are interconvertible via a low energy barrier, tautomeric isomerism ('tautomerism') can occur. This can take the form of proton tautomerism in active ingredients as disclosed herein containing, for example, an imino, keto, or oxime group, or so-called valence tautomerism in compounds which contain an aromatic moiety. It follows that a single compound may exhibit more than one type of isomerism.

Included within the scope of the present disclosure are all stereoisomers, geometric isomers and tautomeric forms of the active ingredients as disclosed herein, including compounds exhibiting more than one type of isomerism, and mixtures of one or more thereof. Also included are acid addition or base salts wherein the counterion is optically active, for example, d-lactate or 1-lysine, or racemic, for example, dl-tartrate or dl-arginine.

Cis/trans isomers may be separated by conventional techniques well known to those skilled in the art, for example, chromatography and fractional crystallisation.

Conventional techniques for the preparation/isolation of individual enantiomers include chiral synthesis from a suitable optically pure precursor or resolution of the racemate (or the racemate of a salt or derivative) using, for example, chiral high pressure liquid chromatography (HPLC).

Alternatively, the racemate (or a racemic precursor) may be reacted with a suitable optically active compound, for example, an alcohol, or, in the case where the active ingredients as disclosed herein contains an acidic or basic moiety, a base or acid such as 1-phenylethylamine or tartaric acid. The resulting diastereomeric mixture may be separated by chromatography and/or fractional crystallization and one or both of the diastereoisomers converted to the corresponding pure enantiomer(s) by means well known to a skilled person.

Chiral compounds of the invention (and chiral precursors thereof) may be obtained in enantiomerically-enriched form using chromatography, typically HPLC, on an asymmetric resin with a mobile phase consisting of a hydrocarbon, typically heptane or hexane, containing from 0 to about 50% by volume of isopropanol, typically from about 2% to about 20%, and from 0 to about 5% by volume of an alkylamine, typically about 0.1% diethylamine. Concentration of the eluate affords the enriched mixture.

Stereoisomeric conglomerates may be separated by conventional techniques known to those skilled in the art - see, for example, Stereochemistry of Organic Compounds by E. L. Eliel and S. H. Wilen (Wiley, New York, 1994), which is incorporated herein in its entirety by reference.

The present invention includes all pharmaceutically acceptable isotopically-labelled compounds of active ingredients as disclosed herein wherein one or more atoms are replaced by atoms having the same atomic number, but an atomic mass or mass number different from the atomic mass or mass number which predominates in nature.

Examples of isotopes suitable for inclusion in the compounds of the invention include, but are not limited to, isotopes of hydrogen, such as 2H and 3H, carbon, such as 11C, 13C and 14C, chlorine, such as 36Cl, fluorine, such as 18F, iodine, such as 1231 and 125I, nitrogen, such as 13N and 15N, oxygen, such as 150, 170 and 180, phosphorus, such as 32P, and sulphur, such as 35S.

Certain isotopically-labelled active ingredients as disclosed herein, for example, those incorporating a radioactive isotope, are useful in drug and/or substrate tissue distribution studies. The radioactive isotopes tritium, i.e. 3H, and carbon-14, i.e. 14C, are particularly useful for this purpose in view of their ease of incorporation and ready means of detection.

Substitution with heavier isotopes such as deuterium, i.e. 2H, may afford certain therapeutic advantages resulting from greater metabolic stability, for example, increased in vivo half-life or reduced dosage requirements, and hence may be preferred in some circumstances.

Substitution with positron emitting isotopes, such as 11C, 18F, 150 and 13N, can be useful in Positron Emission Topography (PET) studies for examining substrate receptor occupancy.

Isotopically-labeled active ingredients as disclosed herein can generally be prepared by conventional techniques known to those skilled in the art or by processes analogous to those described in the accompanying Examples using an appropriate isotopically-labeled reagent in place of the non-labeled reagent previously employed.

It is believed that free radicals and hypoxia can increase the damage to mitochondrial DNA and produce undesirable changes in epigenetics related to risk of cancer growth and metastasis through hypoxia induced factor one and VEGF. Hypoxia is a common characteristic of locally advanced solid tumors that has been associated with diminished therapeutic response and, more recently, with malignant progression. Emerging evidence indicates that the effect of hypoxia on malignant progression is mediated by a series of hypoxia-induced proteomic and genomic changes activating angiogenesis, anaerobic metabolism, and other processes that enable tumor cells to survive or escape their oxygen deficient environment. The transcription factor hypoxia-inducible factor 1 ("HIF-1") is a regulator of tumor cell adaptation to hypoxic stress. Tumor cells with proteomic and genomic changes favoring survival under hypoxic conditions will proliferate, thereby further aggravating the hypoxia. The selection and expansion of new (and more aggressive) clones, which eventually become the dominant tumor cell type, lead to the establishment of a vicious circle of hypoxia and malignant progression. Hypoxia tends to increase tissue factor expression by malignant cells which enhances tumor cell-platelet binding and hematogenous metastasis. Hypoxia, whatever its duration, increases the nuclear content of HIF-1 as well as the mRNA levels of erythropoietin and VEGF. HIF-1 plays an important role in solid tumor cell growth and survival. Overexpression of HIF-1 alpha has been demonstrated in many human tumors and predicts a poor response to chemoradiotherapy.

In that regard, it is believed that hyperbaric oxygen therapy (HBOT) can play a positive role in certain malignancies and increase quality of life in patient when used along with chemotherapy, inhibit the certain cancer genes and tumor growth in vitro, and reduce the tumor burden and restricts the growth of large tumor cell colonies. It is possible that this effect is through lowering the HIF-1 which can change the expression in the VEGF gene subsequently involved in tumor metastasis. VEGF is an initiator of tumor angiogenesis. Furthermore, it is believed that VEGF expression is potentiated by hypoxia and that the potentiation of VEGF production in hypoxic areas of solid tumors contributes to VEGF-driven tumor angiogenesis.

It is believed that free radical related lesions that do not cause cell death can stimulate the development of cancer and can promote cancer growth, and metastasis. Reactive oxygen species generate mitochondrial DNA mutation and up regulates HIF-I, therefore reducing oxidative damage is beneficial.

There are available treatments that effectively reduce free radical production and cellular damage. These treatments can potentially modify the epigenetics and increase the effectiveness of other treatments such as DCA and 3 BP. As a result combining HBOT with the formulations and methods disclosed herein would be beneficial.

The present invention also contemplates the use of treatment with hyperbaric oxygen either before or after the administration of any epigenetic modifier, either singly or combined or co-administered with another epigenetic modifier. In general, hyperbaric oxygenation, or hyperbaric oxygen therapy, is a treatment in which an individual is exposed to an environment of increased oxygen at ambient pressure greater than one atmosphere for a predetermined period of time. Hyperbaric oxygen therapy has been approved to treat many conditions, including embolisms, carbon monoxide poisoning, crush injuries, decompression sickness, anemia, and bone infections. Hyperbaric oxygen therapy and various hyperbaric treatment equipment (such as hyperbaric chambers) are generally known in the art and described in various patents, such as US Patent No. 5,865,722, which is incorporated herein in its entirety by reference. Any suitable hyperbaric equipment or chamber may be used in the present invention. The one or more epigenetic modifiers may be administered before or after treatment of hyperbaric oxygen, such as for example, in a hyperbaric chamber. The hyperbaric oxygen treatment may occur within about 24 hours before or after the administration of any epigenetic modifier, between about five (5) minutes and about ninety (90) minutes before or after the administration of any epigenetic modifier, or between about thirty (30) minutes and about three (3) hours before or after the administration of any epigenetic modifier.

### EXAMPLES

### Example 1: Formulations, Kits, and Administration

In various embodiments, a formulation is provided comprising SPB and quercetin. The formulation may be in a combined form. The formulation uses a saline medium, wherein there is about 0.5 g to about 1.0 g of quercetin and about 5.0 g to about 10.0 g of the SPB. In further embodiments, D5 saline is used in lieu of normal saline medium. In further embodiments, the formulation further comprises an antioxidant.

In various embodiments, a formulation is provided comprising LA and quercetin. The formulation may be in a combined form. The formulation uses a saline medium, wherein there is about 0.5 g to about 1.5 g of quercetin and about 200 mg to about 1000 mg of the LA. In further embodiments, D5 saline is used in lieu of normal saline medium.

In various embodiments, a formulation is provided comprising LA and SPB. The formulation may be in a combined form. The formulation uses a saline medium, wherein there is about 200 mg to about 1000 mg of the LA and about 1.0 g to about 10.0 g of the SPB. In further embodiments, D5 saline is used in lieu of normal saline medium.

In various embodiments, a formulation is provided comprising SPB, quercetin, and a glycolytic inhibitor. In various embodiments, the glycolytic inhibitor comprises at least one of 3-BP, DCA, and octreotide. The formulation may be in a combined form. The formulation uses a saline medium, wherein there is 0.5 g to about 1.5 g of quercetin, about 1.0 g to about 10.0 g of the SPB. In further embodiments, D5 saline is used in lieu of normal saline medium.

In various embodiments, a formulation is provided comprising green tea extract (e.g., EGCG) and SPB. The formulation is in a combined form. The formulation uses a saline medium, wherein there is about 100 mg to about 1.5 g green tea extract and about 1.0 g to about 10.0 g SPB.

Any formulation in accordance with various embodiments may be packaged in a kit, as described herein. In addition, active ingredients disclosed herein may be co-administered or temporally closely spaced administered as described above.

### Example 2: Hyperbaric Oxygen Therapy (HBOT)

After administration or co-administration of any active ingredient as disclosed herein, HBOT may be administered. In various embodiments, a patient is subjected to HBOT between about 5 minutes and about 90 minutes after the administering of any active ingredient as disclosed herein. The HBOT environment comprises an atmosphere of at least above 95% O2 at a pressure of about 0.5 atm to about 2.5 atm, and more preferably from about 1.5 atm to about 2 atm. The HBOT occurs for between about thirty minutes and about three hours.

### Example 3: Studies

Consistent with that disclosed herein, various studies were conducted using targeted therapies to reduce anabolic glycolysis in patients with cancer along with epigenetic modifier treatment using HDACIs and hyperbaric oxygen. These treatments were shown to increase quality of life and can improve the patient survival. More particularly, an integrative cancer care/approach was undertaken to treat patients who referred for such intervention voluntarily.

Study I: Forty (40) patient charts were selected randomly and reviewed. The inclusion criteria were diagnosis of cancer. No patients were excluded. Patients were aged 27 to 83 years. All were diagnosed by their oncologist/physician and were offered standard conventional treatment of surgery, traditional chemotherapy or radiation. Out of 40 patients 20 of them refused standard care or there was no conventional option available for them due to severity of the disease. Out of 40 patients, 23 of them had advanced stage disease with micro or macro multiple metastasis at the time of referral, before starting the treatment. 19 of these patients (47 percent) had already been treated with multiple chemotherapy agents unsuccessfully and had progression or recurrence of disease manifested by their tumor markers or scans.

The patients were managed based on unique developed protocols that were designed in correlation with available research studies and clinical trials that implicate using specific natural and synthetic IV therapies. IV therapies are targeted at epigenetic level and consist of antioxidants, quercetin, DCA, sodium phenyl butyrate, and lipoic acid separately or in combination. All patients received one or more of such treatments. Doses of each treatment remained same or close on each treatment, quercetin was given intravenously at the dose about 0.5 to about 1.5 gram (50mg/ml). When administered, SPB was dosed at about 1.0 g to about 10.0 g (25 to 50 ml of 200 mg/ml. When administered, DCA was dosed at about 500 mg to about 6 gram (maximum 100/kg). When administered, lipoic acid was given at about 200 mg to about 1000 mg. Hyperbaric oxygen treatment was applied, with standard 1.5 to 2.0 atmosphere pressure for 45-90 minutes (average 60 minutes) on each session. When administered octreotide was given subcutaneously at about 50 mcgs to about 400 mcgs.

All patients started the program after educating them about their possible options of conventional and non-conventional treatments and consents obtained. The progression of disease was measures during the course of treatment through Tumor markers, Imaging studies and markers for cancer growth, necrosis, LDH, and inflammation, CRP, as well as the Natural killer cell activity or lymphocyte count and Circulatory tumor cells.

The following results were obtained during or after completing the course of therapy:
1) Subjective Increase in QOL (increase energy level, less pain scores and elevation in mood: 100 percent
2) Immunological response: Increase in Natural Killer (NK) cell activity or white blood cell (WBC) count: 35% of patients had initial low NK/WBC, all these patients have increased NK activity after therapy
3) Potential decrease in tumor activity by measuring LDH: 40 percent of patients had high LDH, ALL these patients have shown decreased LDH after the therapy
4) Response in Tumor markers, enough to qualify for clinical response: 50 percent
5) Shrinkage of tumor in radiographic studies: 35 percent
6) Decrease in CRP (correlation with improved survival): 23 percent
7) Decrease in IgF-1: 12 percent of these patients had increased IgF-1, suggested to correlate with prognosis in literature. All these patients had improved IgF-1 after the treatment

Since patients with cancer may have significant stratifying confounders in selecting their control group, we used each patient's pre interventional status as the control arm. Patients other stratifying confounders did not change during the study.

Analysis of the results from Study I:
1) These data reveals superior response in the group of patients compared to the controls. In 47 percent of patients treated there was no conventional option available at the time of referral. In this group, results are far better compared to conventional modalities of treatment.
2) Patients who received both HBOT and IV therapies did better as far as their imaging, their quality of life and tumor shrinkage as well as controlling their tumor markers than the ones who did the IV therapies only.
3) Patients with stage four terminal disease receiving the above program, exceeded response beyond the standard of care expectations, and the patients who did receive chemotherapy concurrently with above targeted therapies had significant improvement in quality of life and chemotherapy response.

In a further study, 45 patient charts were selected and reviewed retrospectively. The inclusion criteria were diagnosis of cancer, and receiving minimum of two weeks of treatments per protocol. No patients were excluded. Patients were aged 27 to 83 years. All were diagnosed by their oncologist/physician and were offered standard conventional treatment of surgery, traditional chemotherapy or radiation. Out of 45 patients, 25 of them refused conventional chemotherapy or there was no conventional option available for them due to severity of the disease and failure to respond to the standard of care.

Study II: Out of 45 patients, 36 of them (80 percent) were at stage four, and had advanced disease with micro or macro multiple metastasis at the time of referral, before starting the treatment.

25 of these patients (55 percent) had already been treated with standard of care including multiple chemotherapy agents unsuccessfully and had relapse, progression or recurrence of disease manifested by their tumor markers and scans.

The patients were managed based on unique developed protocols that were designed in correlation with available research studies and clinical trials that implicate using specific natural and synthetic IV therapies in combination. IV therapies are targeted at epigenetic level. Hyperbaric oxygen treatment was applied to some of the patients as well, with standard 1.5 to 2.0 atmosphere pressure for 45-90 minutes (average 60 minutes) on each session.

All patients started the program after educating them about their possible options of conventional and non-conventional treatments and consents obtained. The progression of disease was measures during or after the course of treatment through Tumor markers, Imaging studies and markers for cancer growth, necrosis, LDH, and inflammation, CRP, as well as the Natural killer cell activity or lymphocyte count and Circulatory tumor cells.

The following results were obtained during or after completing the course of therapy:
1) Subjective Increase in QOL (increased energy level and function, weight gain, improved pain scores): 98 percent (one patient had line infection treated with antibiotics, and 5 patients had minor reactions requiring anti histamines)
2) Immunological response: Increase in Natural Killer cell activity: 19 patients (42 percent) had initial low natural killer cell activity measured, indicating low immune function. Twelve (12) of 19 were improved after the treatment. One stayed the same and two had lower activity. 3 patients did not follow for their post treatment.
3) Potential decrease in tumor activity by measuring LDH: 42 patients had their LDH measured. (Three patient had unknown LDH) 20 patients (44 percent) had initial high LDH number, 18 of them (90 percent) showed decreased LDH after the therapy.
4) Response in Tumor markers, indicating clinical response: 14 patients had normal tumor markers or there was no tumor marker correlated with their disease. Out of the rest, (31 patients) with high tumor markers, 27 patients (87 percent) had decreased tumor markers after the course of treatment. In two patients, it fluctuated and in one it was stable.
5) Shrinkage of tumor in radiographic/ imaging studies: 25 patients had imaging reports to follow for their condition. The other 20 had no imaging or it was not applicable or relevant. Out of 25 patients, 19 patients (76 percent) had positive response in their scans after the treatments. In seven patients, the results were mixed or stable. Two cases did progress, one after initial response.
6) Decrease in CRP (correlation with improved survival): 22 patients had their CRP elevated. 23 patients had their CRP at normal range or it was not checked. 17 patients had increased C reactive protein. Sixteen (16) patients responded by decreased CRP. One increased.
7) Decrease in IgF-1: 38 patients had their insulin like growth factor checked. 10 patients had increased levels before starting the therapy. All these patients (100 percent) responded by decreased levels after the treatment, suggested to correlate with prognosis in literature. All these patients had improved IgF-1 after the treatment to normal range.
8) Decrease in VEGF: 20 patients had their vascular endothelial growth factor (VEGF) checked. We found four patients with increased levels correlating with higher risk of metastasis. All four had decreased VEGF after the treatments.

Since patients with cancer may have significant stratifying confounders in selecting their control group, we used each patient's pre interventional status as the control arm. Patients other stratifying confounders did not change during the study.

Analysis of the results from Study II:
1) These data reveals superior response in the group of patients compared to the controls. In 47 percent of patients treated, there was no conventional option available at the time of referral. In this group, the results are far better compared to conventional modalities of treatment, of which there are no treatment options available.
2) Patients who received both HBOT and IV therapies did better as far as their imaging, their quality of life and tumor shrinkage as well as controlling their tumor markers than the ones who did the IV therapies only.
3) Patients with stage four terminal disease receiving the above program, exceeded response beyond the standard of care expectations, and the patients who did receive chemotherapy concurrently with above targeted therapies had significant improvement in quality of life and chemotherapy response.

As shown, the use of epigenetic modifiers increased cancer survival as well as quality of life in a number of patients. The above described modality of care was found to be superior to conventional standards of care.

Further aspects of the invention:

## Claims

1. A pharmaceutical formulation for the prophylaxis or treatment of cancer, comprising two or more epigenetic modifiers.

2. The pharmaceutical formulation of aspect 1, wherein the two or more epigenetic modifiers are histone deacetylase inhibitors or demethylating agents.

3. The pharmaceutical formulation of aspect 1, wherein the two or more epigenetic modifiers are selected from a group comprising sodium phenyl butyrate (SPB), lipoic acid (LA), quercetin, valproic acid, hydralazine, bactrim, green tea extract, epigallocathechin gallate, curcumin, sulforphane and allicin/ diallyl disulfide.

4. The pharmaceutical formulation of aspect 3, comprising sodium phenyl butyrate (SPB) and quercetin,

5. The pharmaceutical formulation of aspect 4, wherein the pharmaceutical formulation is in a unit dose comprising about 1.0 g to about 10.0 g of sodium phenyl butyrate (SPB) and about 0.5 g to about 1.5 g of the quercetin.

6. The pharmaceutical formulation of aspect 3, comprising quercetin and lipoic acid (LA).

7. The pharmaceutical formulation of aspect 6, wherein the pharmaceutical formulation is in a unit dose comprising about 0.5 g to about 1.5 g of quercetin and about 200 mg to about 1000 mg of the lipoic acid (LA).

8. The pharmaceutical formulation of aspect 3, comprising lipoic acid (LA) and sodium phenyl butyrate (SPB).

9. The pharmaceutical formulation of aspect 8, wherein the pharmaceutical formulation is in a unit dose comprising about 200 mg to about 1000 mg of the lipoic acid (LA) and about 1.0 g to about 10.0 g of the sodium phenyl butyrate (SPB).

10. The pharmaceutical formulation of aspect 3, comprising green tea extract and sodium phenyl butyrate (SPB).

11. The pharmaceutical formulation of aspect 10, wherein the pharmaceutical formulation is in a unit dose comprising about 100 mg to about 1.5 g green tea extract and about 1.0 g to about 10.0 g sodium phenyl butyrate (SPB).

12. The pharmaceutical formulation of aspect 1, further comprising one or more oxidants or antioxidants.

13. The pharmaceutical formulation of aspect 12, wherein the one or more oxidants or antioxidants are selected from a group comprising vitamin C, germanium, L carnitine, taurine, gluthatione, lysine proline, hydrogen peroxide (H202), and dimethyl sulfoxide (DMSO).

14. The pharmaceutical formulation of aspect 1, further comprising one or more pharmaceutically acceptable excipients, diluents or carriers.

15. The pharmaceutical formulation of aspect 14, further comprising at least one of normal saline and D5 saline.

16. The pharmaceutical formulation of aspect 1, wherein the pharmaceutical formulation is suitable for intravenous injection into a human.

17. The pharmaceutical formulation of aspect 1, further comprising one or more glycolytic inhibitors.

18. The pharmaceutical formulation of aspect 17, wherein the one or more glycolytic inhibitors are selected from a group comprising dichloroacetic acid, octreotide, and 2 deoxy glucose (2DG).

19. A unit dose of a pharmaceutical formulation for treatment of cancer comprising two or more epigenetic modifiers in a combined form, wherein the epigenetic modifiers are present in a dosage sufficient to cause tumor response in a human as measured by laboratory and/or radiologic studies after administration of between about 1 unit doses and about 60 unit doses.

20. A unit dose of a pharmaceutical formulation for treatment of cancer comprising two or more epigenetic modifiers in a combined form, wherein the epigenetic modifiers are present in a dosage sufficient to cause increase in immune system measured by increase in white blood count (WBC) and/ or natural killer (NK) cell activity in a human after administration of between about 1 unit dose and about 60 unit doses.

21. A kit comprising:
a unit dose of a pharmaceutical formulation for treatment of cancer comprising two or more epigenetic modifiers;
a container wherein the unit dose is at least partially contained.

22. The kit of aspect 21, wherein the two or more epigenetic modifiers comprise demethylating agents or histone deacetylase inhibitors (HDACI).

23. The kit of aspect 21, wherein the epigenetic modifiers are selected from a group comprising sodium phenyl butyrate (SDB), lipoic acid (LA), quercetin, valproic acid, hydralazine, bactrim, green tea extract, curcumin, sulforphane and allicin/ diallyl disulfide.

24. The kit of aspect 21, further comprising a first subcontainer comprising a first epigenetic modifier and a second subcontainer comprising a second epigenetic modifier.

25. The kit of aspect 21, further comprising a subcontainer comprising a first epigenetic modifier and a second epigenetic modifier in combined form.

26. The kit of aspect 21, further comprising a memory aid for guidance in administration of the unit dose.

27. A method of treatment comprising:
administering one or more epigenetic modifiers to a mammal; and
subjecting the mammal to a hyperbaric oxygen environment.

28. The method of aspect 27, wherein the hyperbaric oxygen environment comprises an atmosphere of above about 95% 02.

29. The method of aspect 27, wherein the subjecting occurs within about twenty-four hours of the administering.

30. The method of aspect 27, wherein the subjecting occurs between about five (5) minutes and about ninety (90) minutes before or after the administering.

31. The method of aspect 27, wherein the subjecting occurs for between about thirty (30) minutes and about three (3) hours before or after the administering.

32. The method of aspect 27, wherein the one or more epigenetic modifiers comprises one or more demethylating agents or histone deacetylase inhibitors (HDACI).

33. The method of aspect 27, wherein the one or more epigenetic modifiers are selected from a group comprising sodium phenyl butyrate (SPB), lipoic acid (LA), quercetin, valproic acid, hydralazine, bactrim, green tea extract, curcumin, sulforphane and allicin/ diallyl disulfide.

34. The method of aspect 33, wherein the one or more epigenetic modifiers comprise sodium phenyl butyrate (SPB).

35. The method of aspect 33, wherein the one or more epigenetic modifiers comprise lipoic acid (LA).

36. The method of aspect 33, wherein the one or more epigenetic modifiers comprise quercetin.

37. The method of aspect 33, wherein the one or more epigenetic modifiers comprise quercetin and sodium phenyl butyrate (SPB).

38. The method of aspect 37, wherein the administering comprises:
administering quercetin intravenously at a dose of about 0.5 g to about 1.5 g in normal saline; and
administering sodium phenyl butyrate (SPB) intravenously at a dose of about 1.0 g to about 10.0 g in normal saline.

39. The method of aspect 33, wherein the one or more epigenetic modifiers comprise quercetin and lipoic acid (LA).

40. The method of aspect 39, wherein the administering comprises:
administering quercetin intravenously at a dose of about 0.5 g to about 1.5 g in normal saline; and
administering lipoic acid (LA) intravenously at a dose of about 200 mg to about 1000 mg in normal saline.

41. The method of aspect 33, wherein the one or more epigenetic modifiers comprise lipoic acid (LA) and sodium phenyl butyrate (SPB),

42. The method of aspect 41, wherein the administering comprises:
administering lipoic acid (LA) intravenously at a dose of about 200 mg to about 1000 mg in normal saline; and
administering sodium phenyl butyrate (SPB) intravenously at a dose of about 1,0 g to about 10.0 gin normal saline.

43. The method of aspect 33, wherein the epigenetic modifiers comprise green tea extract and sodium phenyl butyrate (SPB).

44. The method of aspect 43, wherein the administering comprises:
administering the green tea extract intravenously at a dose of about 100 mg to about 1.5 g in normal saline; and
administering the sodium phenyl butyrate intravenously at a dose of about 1.0 g to about 10.0 g in normal saline.

45. The method of aspect 33, further comprising administering one or more glycolytic inhibitors to the mammal.

46. The method of aspect 33, further comprising administering one or more oxidants or antioxidants to the mammal.

47. A method of treatment comprising:
administering one or more glycolytic inhibitors to a mammal; and
subjecting the mammal to a hyperbaric oxygen environment.

48. The method of aspect 47, wherein the hyperbaric oxygen environment comprises an atmosphere of above 95% 02.

49. The method of aspects 47, wherein the subjecting occurs within about twenty-four hours of the administering.

50. The method of aspect 47, wherein the subjecting occurs between about five (5) minutes and about ninety (90) minutes before or after the administering.

51. The method of aspect 47, wherein the subjecting occurs for between about thirty (30) minutes and about three (3) hours before or after the administering.

52. The method of aspect 47, wherein the one or more glycolytic inhibitors comprises at least one of dichloroacetic acid, octreotide, and 2 deoxy glucose (2DG).

53. The method of aspect 47, further comprising administering one or more HDACI to the patient.

54. The method of aspect 47, further comprising administering one or more oxidants or antioxidants to the patient.

55. A method of treatment comprising:
administering one or more glycolytic inhibitors to mammal; and
administering one or more epigenetic modifiers to the mammal.

56. The method of aspect 55, wherein the one or more glycolytic inhibitors comprises at least one of dichloroacetic acid, octreotide, and 2 deoxy glucose (2DG).

57. The method of aspect 55, wherein the one or more epigenetic modifiers comprise demethylating agents or histone deacytelase inhibitors (1-IDACI).

58. The method of aspect 55, wherein the one or more epigenetic modifiers are selected from a group comprising sodium phenyl butyrate (SDB), lipoic acid (LA), quercetin, valproic acid, hydralazine, bactrim, green tea extract, epigallo catechin gallate, curcumin, sulforphane and allicin/ diallyl disulfide.

59. The method of aspect 55, further comprising administering one or more oxidants or antioxidants to the patient.
